# EUROPÄISCHE PATENTSCHRIFT

(11) **EP 3 035 051 B1**
(45) Veröffentlichungstag und Bekanntmachung des Hinweises auf die Patenterteilung: **15.04.2020**
(21) Anmeldenummer: 15201284.5
(22) Anmeldetag: 18.12.2015
(51) Int. Cl.: G01N 33/46

(54) **VERFAHREN ZUM UNTERSUCHEN EINES, INSBESONDERE UNTER WASSER BEFINDLICHEN, HOLZKÖRPERS AUF BEFALL MIT HOLZSCHÄDLINGEN**
METHOD FOR EXAMINING A WOOD BODY, IN PARTICULAR ONE UNDER WATER,FOR INFESTATION WITH WOOD PESTS
PROCEDE D'ANALYSE D'UN CORPS EN BOIS SE TROUVANT EN PARTICULIER SOUS L'EAU AFIN DE DECELER DES PARASITES DU BOIS

(30) Priorität: 19.12.2014 DE 102014119274; 19.06.2015 DE 102015109902
(43) Veröffentlichungstag der Anmeldung: 22.06.2016
(73) Patentinhaber: Wießner, Joachim, 49688 Lastrup (DE)
(72) Erfinder: Wießner, Joachim, 49688 Lastrup (DE)
(74) Vertreter: Grabovac, Dalibor

(56) Entgegenhaltungen:
- DE-A1- 2 142 164
- DE-A1- 3 501 841
- DE-A1- 4 122 494
- DE-A1- 10 200 919
- DE-A1- 19 617 307
- DE-A1- 19 934 615
- DE-A1-102009 013 069
- DE-C2- 2 912 605
- DE-U- 1 816 498
- US-A1- 2013 104 634
- ZYCHA H ET AL: "Erfahrungen mit einem Geraet zur Faeuleermittlung an stehenden Staemmen", FORSTWISSENSCHAFTLICHES CENTRALBLATT, PAREY, DE, Bd. 81, 1. Januar 1962 (1962-01-01), Seiten 222-230, XP009189671, ISSN: 0015-8003

## Beschreibung

Die Erfindung betrifft ein Verfahren zum Untersuchen eines unter Wasser befindlichen länglichen Holzkörpers auf Befall mit Holzschädlingen, insbesondere im Meer lebender Holzschädlinge, und/oder zum Ermitteln eines Ausmaßes eines Befalls mit Holzschädlingen, insbesondere im Meer lebender Holzschädlinge, an einem Holzkörper.

Die Erfindung betrifft außerdem ein Set zur Verwendung bei der Ausführung eines solchen Verfahrens.

Es ist bekannt, Bauwerke oder Bäume auf der Basis der sogenannten Bohrwiderstandsmeßmethode auf Schädlingsbefall zu untersuchen. Hierbei wird eine Bohrnadel drehend mit einem speziellen Bohrgerät in das zu untersuchende Material vorgetrieben, wobei der von dem angebohrten Objekt der Bohrnadel entgegengesetzte Widerstand in Abhängigkeit von der Eindringtiefe gemessen wird. Die auf diese Weise ermittelten Messwerte erlauben einen Rückschluss auf die Dichte des durchbohrten Materials. Insbesondere ist es auf diese Weise möglich, Hohlräume zu erkennen. Es ist auch möglich, eine entsprechende Messung beim Widerherausziehen der Bohrnadel aus dem entstandenen Bohrkanal durchzuführen. Allerdings sind die hierbei ermittelten Messwerte in aller Regel nicht so aussagekräftig, wie die beim Eindringen in den Holzkörper ermittelten Messwerte. Der Widerstand kann indirekt beispielsweise durch Messung der Leistungsaufnahme des die Bohrnadel drehenden und/oder des die Bohrnadel voranschiebenden Motors gemessen werden.

Aus DE 10 2009 013 069 A1 ist ein Verfahren zur Untersuchung der Beschaffenheit von säulenförmigen oder zylindrischen Abschnitten von Objekten bekannt. Es umfasst das Anordnen einer Mehrzahl von Sensoren zur Aufnahme von Drucksignalen an einem Umfang des säulenförmigen oder zylindrischen Abschnitts des Objekts, sowie das diskontinuierliche Einführen einer Bohrnadel eines Bohrgerätes unter Durchführung einer Bohrwiderstandsmessung bis zu einer vorbestimmten Eindringtiefe. Das diskontinuierliche Einführen umfasst ein Impuls induzierendes Anhalten, gefolgt von einem Wiederanfahren der Bohrnadel. Die von der Bohrnadel induzierten Impulse erzeugen hierbei Impulswellen, deren sich zum Umfang des Objekts ausbreitende Anteile von den Sensoren detektiert und einer Impulslaufzeitmessung zugeführt werden. Dieses Verfahren ist zur Anwendung unter Wasser ungeeignet.

Aus EP 0 977 653 B1 ist ein Bohrwiderstandsmessgerät bekannt, das aus einem Bohraufsatz und einem Antriebsaggregat besteht. Das Bohrwiderstandsmessgerät dient zur Ermittlung des inneren Zustands und/oder der Dichte an Bäumen oder hölzernen Bauelementen und weist eine dünne ein- oder mehrmals abgestützte Bohrnadel, sowie Mittel zum Aufzeichnen und/oder Mitteln zum elektronischen Speichern des über die Bohrnadel wirkenden Drehmoments (Bohrwiderstand) und der Eindringtiefe der Bohrnadel auf. Um möglichst kleine räumliche Ausmaße und möglichst geringes Gewicht bei gleichzeitig sicherer Handhabung zu erreichen, ist unter anderem vorgesehen, dass das Antriebsaggregat die Bohrnadel und einen Schlitten, in dem die Bohrnadel drehbar gelagert ist, über ein Wechselgetriebe und ein Planetengetriebe eine Gewindespindel antreibt.

Aus DE 35 01 841 A1 sind Verfahren und eine Vorrichtung bekannt zur Feststellung des Gesundheitszustandes von Bäumen an Straßen, in Plantagen, in Parks oder in Wäldern, sowie zur Überprüfung eventueller Beschädigungen durch pflanzlichen oder tierischen Schädlingsbefall von hölzernen Masten oder Holzbauteilen von Brücken, in Gebäuden oder an Gerüsten. Bei dem Verfahren dringt eine Nadel in das Holz ein, wobei in Abhängigkeit von der Eindringtiefe der Eindringwiderstand gemessen wird.

Aus DE 41 22 494 ist Vorrichtung zur Materialprüfung, insbesondere Holzprüfung, durch Bohr- bzw. Eindringwiderstandsmessung bekannt. Die Vorrichtung hat für den Nadelvorschub einen motorisch angetriebenen Schlitten, der einen die Nadel in Drehung antreibenden Motor trägt. Außerdem ist eine Steuerelektronik einschließlich einer Einrichtung zur Erfassung und Aufzeichnung der Leistungsaufnahme des Nadelantriebs vorhanden.

Aus DE US 2013/0104634 A1 sind ein Verfahren und ein Gerät zur Beschaffenheitsuntersuchung von Holz und gegebenenfalls anderen säulenförmigen oder zylindrischen Abschnitten von Körpern bekannt. Das Gerät umfasst eine Antriebsvorrichtung mit einem Bohrfutter, in das eine Bohrnadel, die durch die Antriebsvorrichtung antreibbar ist, aufgenommen ist.

Aus Zycha et al. "Erfahrungen mit einem Gerät zur Fäuleermittlung an stehenden Stämmen", Forstwissenschaftliches Zentralblatt, Bd. 81, 01.01.1962 ist ein Gerät bekannt, das auf der Anzeige des Einstichwiderstandes einer Stahladel beruht, die über ein Feder- und Hebelsystem in den Stamm eines Baumes gedrückt wird.

Aus DE 196 17 307 A1 ist ein Verfahren zum Prüfen des inneren Zustandes von Bäumen und Holzbauteilen durch das mechanische, maschinelle und spanlose Ausstanzen und Ziehen eines Holzkernes bekannt. Bei dem Verfahren wird ein dünnwandiges Rohr mit einem Rohrkopf mit größerem Außendurchmesser und kleinerem Innendurchmesser als das restliche Rohr mit hoher Drehzahl in das Holz gedrückt.

Insbesondere in den letzten Jahren haben die Schäden an unterwasserverbauten Hölzern durch Holzschädlinge, wie beispielsweise die Bohrmuschel, oder die Bohrassel sehr zugenommen. Die unter dem lateinischen Namen "Teredo navalis" bekannte Bohrmuschel wird auch Schiffsbohrwurm oder Schiffsbohrmuschel genannt. Ein wesentliches Merkmal dieses Schädigers ist, dass er als Larve das Holz erreicht, sich in das Holz einbohrt und den Einbohrgang mit Kalkplättchen verschließt. Von außen bleiben lediglich zwei kleine Atemsiphone sichtbar, während der Holzkörper innen durch die Bohrmuschel zersetzt wird. Es ist durch bloße Inaugenscheinnahme nicht möglich zu beurteilen, ob ein Befall mit dem Holzbohrwurm vorliegt. Insbesondere ist es nicht möglich, allein durch Inaugenscheinnahme festzustellen, welches Ausmaß ein etwaiger Befall aufweist. Die Bohrgänge von Teredo weisen einen Durchmesser von bis zu 1 cm auf. Durchschnittlich weisen die Bohrgänge von Teredo einen Durchmesser von ca. 8 mm auf.

Ein anderer Schädling, der insbesondere unter Wasser verbaute Holzkörper angreift, ist die Bohrassel (Limnoria lignorum). Diese ist im Vergleich zur Bohrmuschel eher klein (bis zu 5 mm lang) und erzeugt Gänge von bis zu 1 mm Durchmesser. Die Bohrassel kommt häufig gemeinsam mit dem Schiffsbohrwurm vor. Die durch die Bohrassel erzeugten Schäden sind bei stärkerem Wellenschlag oftmals auch an der Oberfläche eines Pfahls sichtbar. Dennoch wird die Zerstörungskraft dieses Schädlings zumeist unterschätzt.

Aus DE 10 31 34 A1 ist ein Verfahren und eine Vorrichtung zur Kennzeichnung und Bewertung von Materialeigenschaften von Prüflingen bekannt. Hierbei wird mit einer Bohrnadel beispielsweise ein Bauwerk angebohrt, wobei ein Antrieb die Bohrnadel dreht und aus einem Bohrgerät in das zu untersuchende Material schiebt. Gemessen und aufgezeichnet werden dabei der Leistungs- beziehungsweise Stromverbrauch des Bohrwerkzeugantriebs beim Eindringen und/oder Herausziehen aus dem Prüfling. Um Messungen im strömenden Regen, beispielsweise in tropischen Wäldern, oder Prüfungen von beispielsweise hölzernen Brücken, Fundamenten unter Wasser vornehmen zu können, wird in DE 10 31 34 A1 vorgeschlagen, dass das Bohrgerät nur eine Öffnung aufweist, durch die das Bohrwerkzeug drehend oder stehend ein- und ausfährt, wobei im Inneren des Bohrgerätes ein erhöhter Gasinnendruck aufgebaut wird, der dem gleichzeitig gemessenen Außendruck, vorzugsweise durch Wasser, so entspricht, dass kein Wasser eintreten kann. Eine Verwendung einer solchen Vorrichtung zum Untersuchen eines Holzkörpers auf Befall mit Holzschädlingen, insbesondere auf Befall mit im Meer lebenden Holzschädlingen, offenbart diese Druckschrift nicht.

Es ist daher die Aufgabe der vorliegenden Erfindung, ein Verfahren anzugeben, das es ermöglicht, zuverlässig feststellen zu können, ob und/oder in welchem Umfang an einem, insbesondere unter Wasser befindlichen, Holzkörper ein Befall mit Holzschädlingen, insbesondere im Meer lebender Holzschädlinge, vorliegt.

Die Aufgabe wird durch ein Verfahren gelöst, das dadurch gekennzeichnet ist, dass mit einem Bohrwiederstandmessgerät mehrere Untersuchungsbohrungen durchgeführt werden, bei denen der einer Bohrnadel von dem Holzkörper entgegengesetzte Widerstand in Abhängigkeit von der Eindringtiefe beim Eindringen der Bohrnadel in den Holzkörper und/oder beim Herausziehen der Bohrnadel aus dem Holzkörper gemessen wird, und dass die Bohrkanäle in unterschiedlichen zueinander parallelen Ebenen Verlaufen, wobei die Ebenen senkrecht zu einer Längserstreckungsachse des länglichen Holzkörpers angeordnet sind, und dass
a. die Untersuchungsbohrungen bei einem kreiszylinderförmigen oder halbkreiszylinderförmigen Holzkörper derart durchgeführt werden, dass die Anfänge der Bohrkanäle entlang einer Helixkurve angeordnet sind, und/oder dass
b. eine Bohrschablone an dem Holzkörper befestigt wird, die die Anfänge der Bohrkanäle definiert, wobei die Bohrschablone ein Justierelement aufweist, das mit einem Justiervorsatz des Bohrwiederstandmessgerätes zusammen wirkt.

Es ist eine weitere Aufgabe der vorliegenden Erfindung, ein Mittel anzugeben, das ein zuverlässiges Untersuchen eines Holzkörpers, insbesondere eines unter Wasser und/oder im Meer befindlichen Holzkörpers, nach dem erfindungsgemäßen Verfahren ermöglicht.

Diese Aufgabe wird durch ein Set mit einem Bohrwiderstandsmessgerät, das eine Bohrnadel aufweist und mit einem Justiervorsatz zum Positionieren und/oder Ausrichten des Bohrwiderstandsmessgeräts relativ zu einem zu untersuchenden Holzkörper, und mit einer Bohrschablone, die ein Justierelement aufweist, wobei der Justiervorsatz dazu ausgebildet ist, mit dem Justierelement der Bohrschablone zusammen zu wirken, gelöst.

Es ist eine weitere Aufgabe der vorliegenden Erfindung, ein Verfahren anzugeben, das es ermöglicht, einen im Wasser befindlichen Holzkörper und/oder im Meer befindlichen Holzkörper, hinsichtlich eines Befalls mit Holzschädlingen zu überwachen.

Diese Aufgabe wird durch ein Verfahren gelöst, das dadurch gekennzeichnet ist, dass zunächst eine Untersuchung, insbesondere nach einem Verfahren nach dem oben genannten Verfahren, durchgeführt wird und anschließend ein Opferholzkörper unter Wasser an dem Holzkörper befestigt wird, der später einmalig oder in regelmäßigen Zeitabständen auf Befall untersucht wird.

Für das in der oben genannten DE 100 31 334 A1 beschriebene Verfahren wird auch der Begriff der Bohrwiderstandsmessung verwendet. Hierbei ist in der Fachwelt bekannt, dass dieses Untersuchungsverfahren, wie alle bohrenden Untersuchungsverfahren, das zu untersuchende Material schädigt.

Darüber hinaus ist es in der Fachwelt als weiterer Nachteil bekannt, dass es sich bei der Durchführung von Einzelmessungen lediglich um eine Stichprobe handelt, die zumeist keine ausreichende Aussage zulässt, wohingegen die Durchführung einer Vielzahl von Bohrungen an unterschiedlichen Stellen zwangsläufig eine Schädigung des Prüflings nach sich führt, die so weit geht, dass der Prüfling hinsichtlich seiner Funktionsfähigkeit, insbesondere hinsichtlich seiner Standfestigkeit, nachhaltig geschädigt ist.

Insbesondere aus diesen Gründen erscheint die Anwendung einer Untersuchungsmethode, die auf der Durchführung von Bohrungen basiert, nicht geeignet zu sein, einen Holzkörper auf Schädlingsbefall zu untersuchen, da die sehr dringende Gefahr besteht, den Holzkörper dermaßen zu schädigen, dass er im Ergebnis ohnehin ausgetauscht werden müsste.

Insbesondere zur Untersuchung von im Wasser, insbesondere im Meerwasser befindlichen Holzkörpern erscheint eine solche Untersuchungsmethode, die auf der Durchführung von Bohrungen basiert, erst recht nicht geeignet, weil ein im Wasser befindlicher Holzkörper, wie beispielsweise ein Pfahl einer Pfahlgründung, nach einer solchen, schädigenden Untersuchung nicht ohne weiteres - oder allenfalls mit einem ganz erheblichen Aufwand - ausgetauscht werden kann.

Insbesondere im Hinblick auf eine Schädigung durch Bohrmuscheln oder Bohrasseln erscheint eine Untersuchungsmethode, bei der Untersuchungsbohrungen durchgeführt werden, ohnehin nicht geeignet, weil die Fraßgänge, deren Durchmesser im Bereich weniger Millimeter liegen, nur dann zuverlässig aufgefunden werden können, wenn die Untersuchungsbohrungen in sehr engem Abstand zueinander ausgeführt werden, was im Hinblick auf die Tragfestigkeit des Holzkörpers aus den oben genannten Gründen unmöglich erscheint.

Wie bereits geschildert, sind die Fraßgänge von Teredo durchschnittlich 8 mm breit. Würde man, um die Fraßgänge zuverlässig aufzufinden, einen Pfahl, insbesondere eine Dalbe, oder einen Pfosten umlaufend im Abstand von 1 cm anbohren und die Bohrnadel jeweils auf der gegenüberliegenden Seite aus dem Holzkörper austreten lassen, dann würde der Holzkörper über den gesamten Querschnitt nahezu abgetrennt. Dies belegt die obige Überlegung, dass die Verwendung der Bohrwiderstandsmeßmethode zum Auffinden derartiger Holzschädlinge nicht tauglich erscheint.

Die vorliegende Erfindung überwindet diese Vorurteile.

Wie im Folgenden an Hand erläutert wird, ist es durchaus möglich, einen Holzkörper zuverlässig dahingehend zu untersuchen, ob ein Befall mit Holzschädlingen vorliegt, und/oder hinsichtlich des Ausmaßes eines Befalls mit Holzschädlingen zu untersuchen; dies insbesondere auch dann, wenn sich der Holzkörper, beispielsweise als Bestandteil einer Pfahlgründung oder als Dalbe, unter Wasser, insbesondere im Meer, befindet.

Insbesondere kann vorteilhaft vorgesehen sein, dass auf der Basis der gemessenen Bohrwiderstandswerte ermittelt wird, ob und/oder in welchem Ausmaß die Tragfähigkeit des untersuchten Holzkörpers durch einen Befall reduziert ist.

In erfindungsgemäßer Weise wurde erkannt, dass es durchaus möglich ist, eine ausreichende Anzahl von Untersuchungsbohrungen derart anzuordnen, dass die Fraßgänge von Holzschädlingen, insbesondere die Fraßgänge von Bohrmuscheln oder Bohrasseln, zuverlässig aufgefunden werden können, ohne dass eine nachhaltige Schädigung des Holzkörpers, insbesondere hinsichtlich seiner Tragfähigkeit, eintritt.

Erfindungsgemäß ist vorgesehen, dass die Untersuchungsbohrungen derart durchgeführt werden, dass die dabei entstehenden Bohrkanäle in unterschiedlichen, zueinander parallelen Ebenen verlaufen.

Je stärker das Ausmaß eines Befalls, beispielsweise eines kreiszylinderförmigen Pfahls, mit Bohrmuscheln ist, umso mehr Fraßgänge finden sich im Kern des Pfahles. Es ist nämlich so, dass die Bohrmuschel zunächst die äußersten Schichten eines Pfahles befällt und sich erst sehr viel später zum Kern hin vorarbeitet. Dies gilt insbesondere auch für die Bohrassel, die das außenliegende Frühholz bevorzugt und zunächst das weiter innen liegende Spätholz stehen lässt, bis außen nicht mehr genügend Nahrung zur Verfügung steht. Wenn nun in unterschiedlichen Ebenen und unterschiedlichen Richtungen, jeweils durch die Mittelachse des Pfahles verlaufend Untersuchungsbohrungen durchgeführt werden, so wird die Tragfähigkeit des Pfahles zum einen nicht merklich geschädigt; zum anderen sind die Bohrgänge der Untersuchungsbohrungen im Bereich des Kernes jedoch so eng zueinander angeordnet, dass ein vorhandener Fraßgang eines Holzschädlings mit äußerst hoher Wahrscheinlichkeit auch aufgefunden wird. Hierbei wird auch ausgenutzt, dass der Kern eines Pfahles zur statischen Biegebelastbarkeit wesentlich weniger beiträgt, als die äußeren Pfahlschichten.

Hierbei wird in vorteilhafter Weise insbesondere ausgenutzt, dass der der Bohrnadel entgegengesetzte Widerstand abnimmt, wenn diese auf einen Fraßgang eines Holzschädlings trifft. Hierbei kann der entgegengesetzte Widerstand beispielsweise indirekt über die elektrische Leistung des die Bohrnadel vorschiebenden und/oder die elektrische Leistung des die Bohrnadel drehenden Motors in Abhängigkeit von der Eindringtiefe gemessen werden. Aus den Messkurven der unterschiedlichen Untersuchungsbohrungen kann dann der Verlauf eines vorhandenen Fraßganges bzw. die Verläufe der vorhandenen Fraßgänge innerhalb des untersuchten Holzkörpers rekonstruiert werden.

Es kann vorteilhaft vorgesehen sein, dass die Untersuchungsbohrungen derart ausgeführt werden, dass die entstehenden Bohrkanäle in unterschiedliche Richtungen verlaufen. Insbesondere kann vorteilhaft vorgesehen sein, dass die Untersuchungsbohrungen derart ausgeführt werden, dass die Projektionen der entstehenden Bohrkanäle auf eine der Ebenen in unterschiedliche Richtungen verlaufen. Auch dies hat den ganz besonderen Vorteil, dass sich in die eingebrachten Bohrkanäle nicht wesentlich auf die Tragfähigkeit des Holzkörpers auswirken. Insbesondere besteht der besondere Vorteil, dass sich die geringe Schädigungswirkung der einzelnen Bohrkanäle nicht kumuliert. Darüber hinaus besteht bei einer derartigen Vorgehensweise der Vorteil, dass etwaig vorhandene Fraßgänge zuverlässiger aufgefunden werden können.

Bei einer ganz besonders vorteilhaften Ausführungsform werden die Untersuchungsbohrungen derart ausgeführt, dass die Projektionen der entstehenden Bohrkanäle auf eine der zueinander parallelen Ebenen sternförmig angeordnet sind. Dies kann beispielsweise dadurch erreicht werden, dass die Untersuchungsbohrungen derart ausgeführt werden, dass die Bohrkanäle durch eine Symmetrieachse und/oder eine Mittelachse, insbesondere eine Rotationsmittelachse, des Holzkörpers verlaufen.

Insbesondere kann vorteilhaft vorgesehen sein, dass die Untersuchungsbohrungen derart ausgeführt werden, dass die Bohrkanäle in Radialrichtung verlaufen. Eine solche Ausführung hat den besonderen Vorteil einer geringen Verringerung der Tragfähigkeit und den weiteren Vorteil, dass etwaig vorhandene Fraßgänge zuverlässig und mit hoher Wahrscheinlichkeit aufgefunden werden können.

Bei einer ganz besonders voreilhaften Ausführung werden die Untersuchungsbohrungen derart ausgeführt, dass die Bohrkanäle vollständig durch den Holzkörper hindurch verlaufen. Sie können insbesondere auch an der gegenüberliegenden Seite aus dem Holzkörper austreten. Auf diese Weise ist es ausreichend, beispielweise bei einem kreiszylinderförmigen Holzkörper, wie beispielsweise einem Pfahl, in Umfangsrichtung lediglich über den halben Umfang Untersuchungsbohrungen, vorzugsweise durch die Mittelachse, durchführen zu müssen, um in der Projektion gesehen den gesamten Querschnitt des Holzkörpers untersuchungsmäßig zu erfassen.

Insbesondere bei einem im Querschnitt kreiszylinderförmigen Holzkörper ist es von besonderem Vorteil, wenn die Untersuchungsbohrungen derart ausgeführt werden, dass die Bohrkanäle senkrecht zur Oberfläche des Holzkörpers und/oder senkrecht zu einer Tangentialebene an den Holzkörper am Ort des Beginns des Bohrkanals, verläuft. Auf diese Weise kann sichergestellt werden, dass die Bohrkanäle durch die Mittelachse des Holzkörpers verlaufen.

Es ist erfindungsgemäß vorgesehen, dass die oben genannten, parallel zueinander ausgerichteten Ebenen, in denen die Bohrkanäle verlaufen, senkrecht zu einer Längserstreckungsachse des Holzkörpers angeordnet sind. Auf diese Weise wird vorteilhaft erreicht, dass die Untersuchungsbohrungen symmetrisch zum Holzkörper ausgeführt werden können und dass die Bohrkanäle, insbesondere bis zum Erreichen der jeweils gegenüberliegenden Seite nicht länger sind, als nötig.

Das Ausführen der Untersuchungsbohrungen derart, dass die Anfänge der Bohrkanäle entlang einer, insbesondere einer entlang der Oberfläche des Holzkörpers verlaufenden Helixkurve, angeordnet sind, hat den ganz besonderen Vorteil, dass von vornherein sichergestellt ist, dass in zueinander parallelen Ebenen jeweils nur eine einzige Untersuchungsbohrung eingebracht wird, wobei die einzelnen Untersuchungsbohrungen in unterschiedliche Richtungen verlaufen.

Bei einem kreiszylinderförmigen Holzkörper, wie beispielsweise einem Pfahl ist auf diese Weise sichergestellt, dass die einzelnen Untersuchungsbohrungen alle durch die Mittelachse des Holzkörpers verlaufen. In der Projektion gesehen sind die Bohrkanäle dann sternförmig angeordnet. Wie weiter unten noch ausführlich beschrieben wird, eignet sich insbesondere bei einer derartigen Vorgehensweise die Verwendung einer Bohrschablone, die vor Durchführung der Untersuchungsbohrungen an dem Holzkörper angebracht wird.

In vorteilhafter Weise können die Untersuchungsbohrungen derart durchgeführt werden, dass die Enden der einzelnen Bohrkanäle entlang einer weiteren Helixkurve, die vorzugsweise über die Oberfläche des Holzkörpers verläuft, angeordnet sind.

Bei einer ganz besonderen Ausführung werden die Untersuchungsbohrungen, insbesondere bei einem kreiszylinderförmigen oder halbzylinderförmigen Holzkörper derart ausgeführt, dass jeder der Bohrkanäle durch jede der beiden Helizes einer Doppelhelix, insbesondere bei einer zum Holzkörper koaxialen Doppelhelix, verläuft.

Insbesondere bei einer solchen Ausführung ist es ausreichend, über einen halben Umfang in den unterschiedlichen Ebenen die Untersuchungsbohrungen einzubringen, sofern die Bohrkanäle soweit geführt werden, dass die Bohrnadel jeweils auf der gegenüberliegenden Seite, insbesondere die weitere Helix schneidend, wieder austreten. Die Bohrkanäle sind dann analog so angeordnet, wie die chemischen Bausteine, die die beiden Stränge der Doppelhelix einer DNA verbinden.

Insbesondere um eine zuverlässige Aussage über das Ausmaß eines Befalls machen zu können, ist es von besonderem Vorteil, wenn die Untersuchungsbohrungen derart ausgeführt werden, dass die Abstände der Anfänge benachbarter Bohrkanäle gleich sind.

Bei einer besonderen Ausführung werden die Untersuchungsbohrungen derart ausgeführt, dass benachbarte Ebenen einen Abstand zueinander aufweisen, der wenigstens dem halben Durchmesser der Bohrnadel und/oder des Bohrkanals entspricht. Alternativ oder zusätzlich kann auch vorgesehen sein, dass die Untersuchungsbohrungen derart ausgeführt werden, dass benachbarte Ebenen, in denen jeweils zumindest ein Bohrkanal verläuft, einen Abstand zueinander aufweisen, der im Bereich des halben bis des doppelten Durchmessers, insbesondere im Bereich des halben bis ganzen Durchmessers, der Bohrnadel und/oder des Bohrkanals liegt.

In besonders vorteilhafter Weise kann vorgesehen sein, dass die Anfänge benachbarter Bohrkanäle jeweils einen Abstand zueinander aufweisen, der im Bereich des halben bis doppelten Durchmessers, insbesondere des halben bis ganzen Durchmessers, der Bohrnadel und/oder des Bohrkanals liegt. Auf diese Weise ist sichergestellt, dass die Bohrkanäle eng genug beieinander liegen, um zuverlässig etwaig vorhandene Fraßgänge aufspüren zu können.

Insbesondere um die Fraßgänge von Bohrmuscheln und/oder von Bohrasseln aufspüren zu können kann vorteilhaft vorgesehen sein, dass für die Untersuchungsbohrungen eine Bohrnadel verwendet wird, deren Bohrspitze einen Durchmesser im Bereich von 2 mm bis 5 mm, insbesondere von 3,5 mm aufweist. Darüber hinaus kann - alternativ oder zusätzlich - vorteilhaft vorgesehen sein, dass die Untersuchungsbohrungen derart ausgeführt werden, dass die Anfänge benachbarter Bohrkanäle einen Abstand zueinander aufweisen, der im Bereich von 0,5 cm bis 1,5 cm liegt oder 1 cm beträgt.

Die Verwendung einer Bohrschablone hat den ganz besonderen Vorteil, dass sichergestellt ist, dass die einzelnen Untersuchungsbohrungen an den richtigen Orten angesetzt werden. Insbesondere wenn der Holzkörper, beispielsweise als Teil einer Pfahlgründung, unter Wasser in Küstennähe aufgestellt ist, ist das Wasser zumeist durch Brandung und/oder Schiffsverkehr derart trüb, dass oftmals nur eine Sichtweite weit unterhalb von 1 m besteht. Ein Taucher, der bestrebt ist, ordnungsgemäße Untersuchungsbohrungen durchzuführen und der oftmals mit zusätzlichen Widrigkeiten, wie Strömungen und/oder niedrigen Temperaturen zu kämpfen hat, kann sich hierbei in ganz besonders vorteilhafter Weise an der Bohrschablone orientieren.

Die Bohrschablone kann auf unterschiedlichste Weise ausgeführt sein. Insbesondere kann die Bohrschablone, wie weiter unten noch ausführlich erläutert ist, aus einem biegsamen Flachmaterial gebildet sein, das flächig anliegend an dem Holzkörper befestigt werden kann.

Sobald die Bohrschablone an dem zu untersuchenden Holzkörper befestigt ist, braucht die die Untersuchung ausführende Person lediglich bei den durch die Bohrschablone definierten Orten eine Untersuchungsbohrung anzusetzen. Beispielsweise kann die Bohrschablone eine Vielzahl von Durchgängen aufweisen, die im Querschnitt so dimensioniert sind, dass die Bohrnadel hindurch gesteckt werden kann und die Bohrnadel bei der durchzuführenden Untersuchungsbohrung in radialer Richtung zuverlässig geführt ist. Vorzugsweise weisen alle Durchgänge denselben Durchmesser und/oder den dem Durchmesser der Bohrnadel entsprechenden Durchmesser auf.

Nachdem ein Holzkörper, insbesondere ein unter Wasser befindlicher Holzkörper, auf Befall mit Holzschädlingen und/oder hinsichtlich des Ausmaßes eines Befalls mit Holzschädlingen untersucht wurde, besteht nach einiger Zeit in aller Regel das Bedürfnis, erneut festzustellen, ob es zu einem Befall gekommen ist beziehungsweise festzustellen, ob sich ein festgestellter Befall verschlimmert hat. In einem solchen Fall könnte natürlich insbesondere das oben beschriebene, erfindungsgemäße Verfahren erneut angewandt werden, wobei jedoch neue Untersuchungsbohrungen durchgeführt werden müssten, um zuverlässige Aussagen treffen zu können. Allerdings besteht hierbei der Nachteil, dass der Holzkörper in seiner Struktur und Tragfähigkeit doch geschädigt wird, wenn erneut und wiederholt jeweils eine Vielzahl von Untersuchungsbohrungen vorgenommen werden.

Aus diesem Grund kann ein Opferholzkörper an dem Holzkörper befestigt werden, der später einmalig oder regelmäßigen Abständen anstelle des Holzkörpers auf Fall untersucht wird. Hierbei wird ausgenutzt, dass die Schädlinge bei ihrem schädlichen Tun nicht zwischen dem Holzkörper und dem daran, vorzugsweise formschlüssig, angebrachten Opferholzkörper unterscheiden. Dies insbesondere dann nicht, wenn die Eigenschaften des Opferholzkörpers, insbesondere hinsichtlich der Holzart oder des Holzalters, mit denen des Holzkörpers übereinstimmen oder diesen zumindest ähneln.

Diese Vorgehensweise hat den ganz besonderen Vorteil, dass bei späteren Befallsuntersuchungen keine weiteren Untersuchungsbohrungen in den Holzkörper eingebracht werden müssen. Vielmehr muss lediglich der Opferholzkörper hinsichtlich seines möglichen Befalls untersucht werden. Dies kann beispielsweise auch nach dem oben beschriebenen Verfahren erfolgen.

Alternativ kann auch in der Weise vorgegangen werden, dass mit Hilfe des Opferholzes regelmäßig überprüft wird, ob ein Befallsdruck herrscht. Wenn bei einer Untersuchung des Opferholzes festgestellt wird, dass kein Befallsdruck herrscht, kann auf eine direkte Untersuchung des Holzkörpers bis zur nächsten Untersuchung des Opferholzes verzichtet werden. Ist jedoch festzustellen, beispielsweise nach zwei weiteren Jahren, dass das Opferholz befallen oder gar erheblich zerstört ist, werden weitere Messungen an dem Holzkörper (insbesondere nach dem oben beschriebenen Verfahren) durchgeführt, um das Befallsausmaß an dem Holzkörper, beispielsweise einem Pfahl unter einer Kajenanlage, direkt feststellen zu können. Hierfür ist es von Vorteil, wenn bei der ersten Untersuchung an dem Holzkörper vermerkt wird, an welcher Stelle, die Untersuchung vorgenommen wurde, damit die Kontrollemessungen der nachfolgenden Untersuchungen in demselben Bereich des Holzkörpers vorgenommen werden. Wenn es sich bei dem Holzkörper beispielsweise um einen Pfahl handelt, ist es von Vorteil zu vermerken, in welcher Pfahlhöhe die erste Untersuchung vorgenommen wurde, damit die nachfolgenden Untersuchungen ungefähr in der gleichen Höhe durchgeführt werden können.

Zur Untersuchung des Opferholzkörpers ist es möglich, diesen von dem Holzkörper, wie beispielsweise einem Pfahl einer Pfahlgründung, zu lösen und zu untersuchen. Bei der Untersuchung kann der Opferholzkörper sogar zerstört werden, weil es in aller Regel ohne weiteres möglich ist, stattdessen einen anderen, neuen Opferholzkörper, an dem zu überwachenden Holzkörper zu befestigen. Alternativ ist es auch möglich, den Opferholzkörper unmittelbar, gegebenenfalls auch unter Wasser, an dem Holzkörper zu untersuchen, ohne diesen von dem Holzkörper zu lösen.

Das erfindungsgemäße Verfahren ist hinsichtlich der Art der Holzkörper, an denen es anwendbar ist, nicht beschränkt. Wie bereits erwähnt können mit dem erfindungsgemäßen Verfahren insbesondere Pfähle, beispielsweise Pfähle von Pfahlgründungen oder Dalben, oder Pfosten zuverlässig untersucht werden. Wie ebenfalls bereits erwähnt, ermöglicht es das erfindungsgemäße Verfahren, die Untersuchung insbesondere hinsichtlich der vorzunehmenden Untersuchungsbohrungen so zu gestalten, dass der Holzkörper nicht oder nicht wesentlich geschwächt wird. Letztlich spielt es für die Anwendbarkeit des Verfahrens keine Rolle, welche äußere Form der Holzkörper hat. Soweit eine Bohrschablone verwendet wird, muss lediglich diese an die Form des zu untersuchenden Holzkörpers angepasst sein. Dies kann insbesondere dadurch erfolgen, dass die Bohrschablone jeweils passend verformt, insbesondere gebogen, wird. Insbesondere kann eine aus einem Blech oder ähnlichem flachen Material hergestellte, elastische Bohrschablone, die eine ebene Ausgangsform aufweist, um einen Pfahl herum gebogen und an dem Pfahl befestigt werden.

Soweit im Rahmen dieser Anmeldung Bohrschablonen für im Querschnitt kreisrunde Holzkörper beschrieben sind, stellt dies keine Einschränkung der Erfindung dar. Vielmehr sind derartige Bohrschablonen auch für andere Holzkörper einsetzbar und/oder an andere Holzkörper anpassbar, die eine andere äußere Form aufweisen.

Wie bereits erwähnt, kann die Bohrschablone insbesondere flach und/oder elastisch biegbar ausgebildet sein, so dass sie leicht an die Außenfläche eines zu untersuchenden Holzkörpers angeschmiegt werden kann.

Vorzugsweise ist die Bohrschablone, insbesondere wenn sie unter Wasser verwendet werden soll, aus einem nicht rostendem Material, wie beispielsweise rostfreiem Blech oder aus Kunststoff hergestellt.

Es ist möglich, die Bohrschablone nach einer abgeschlossenen Untersuchung an dem Holzkörper zu belassen. Vorzugsweise wird die Bohrschablone jedoch entfernt. Ein Entfernen der Bohrschablone hat den besonderen Vorteil, dass nicht für jeden Holzkörper eine eigene Bohrschablone erforderlich ist. Vielmehr kann ein und dieselbe Bohrschablone nacheinander zur Untersuchung an mehreren Holzkörpern eingesetzt werden. Darüber hinaus besteht nicht die Gefahr, dass die Bohrschablone ungewollt, beispielsweise durch Wasserströmungen, losgerissen wird. Insoweit bietet es sich insbesondere an, die Bohrschablone nach der Untersuchung zu entfernen und dann das Opferholz an dem Holzkörper zu befestigen.

Die Bohrschablone kann insbesondere dazu ausgebildet sein, um einen zu untersuchenden Holzkörper, insbesondere wenn es sich um einen im Querschnitt kreisrunden Holzkörper handelt, herum gelegt zu werden.

In jedem Fall ist es von Vorteil, wenn die Bohrschablone an dem zu untersuchenden Holzkörper flächig anliegend befestigt wird. Dies insbesondere in der Weise, dass eine durch Durchführungen der Bohrschablone, die die Anfänge der Bohrkanäle definieren, hindurch gesteckte Bohrnadel unmittelbar auf den Holzkörper trifft.

Bei einer besonderen Ausführungsform der Bohrschablone ist diese dazu ausgebildet und bestimmt, um einen zu untersuchenden Holzkörper, insbesondere flächig anliegend, herum gelegt zu werden, wobei die Bohrschablone für einen Holzkörper mit dem Umfang x ausgelegt ist und die Breite der Bohrschablone wenigstens x/2 beträgt. Eine solche Bohrschablone ermöglicht es, ausreichend viele Untersuchungsbohrungen vornehmen zu können, um den gesamten Querschnitt des Holzkörpers, in der Projektion gesehen, bei der Untersuchung erfassen zu können. Dies insbesondere dann, wenn die Untersuchungsbohrungen in der Weise ausgeführt werden, dass die Bohrkanäle jeweils bis zur gegenüberliegenden Seite reichen und/oder die Bohrnadel jeweils auf der gegenüberliegenden Seite wieder aus dem Holzkörper austritt.

Um zu vermeiden, dass eine auf der gegenüberliegenden Seite des Holzkörpers austretende Bohrnadel die Bohrschablone beschädigt, weist eine besondere Ausführungsform der Bohrschablone in dem diesbezüglich gefährdeten Bereich wenigstens eine Aussparung auf. Insbesondere kann vorteilhaft vorgesehen sein, dass die Aussparung einem der Durchgänge in Bohrrichtung gesehen gegenüberliegend angeordnet ist, wenn die Bohrschablone um einen, insbesondere kreiszylinderförmigen, Holzkörper herum gelegt ist. Das Vorsehen einer Aussparung ist insbesondere dann vorteilhaft, wenn die Bohrschablone im befestigten Zustand sich um mehr als den halben Umfang um den Holzkörper herum legt.

Bei einer ganz besonders voreilhaften Ausführungsform der Bohrschablone sind die Durchgänge, die die Anfänge der Bohrkanäle definieren, entlang einer geraden Linie angeordnet.

Beispielsweise kann die Bohrschablone Durchgänge aufweisen, die, insbesondere wenn die Bohrschablone auf einen ebenen Boden gelegt ist, entlang einer geraden Linie, insbesondere entlang einer Diagonalen, verlaufen. Wird eine solche Ausführung um einen kreiszylinderförmigen Holzkörper herum gelegt und dabei entsprechend gebogen, nehmen die Durchgänge eine Position entlang einer um den kreiszylinderförmigen Holzkörper herum verlaufenden Helixkurve ein.

Bei einer besonderen Ausführungsform weist die Bohrschablone wenigstens ein Befestigungsmittel zum Befestigen der Bohrschablone an einem Holzkörper, insbesondere an einem kreiszylinderförmigen Holzkörper auf. Das Befestigungsmittel kann beispielsweise einen Spanngurt und/oder einen elastischen Gurt aufweisen. Auf diese Weise ist es, auch bei schlechter Sicht unter Wasser, möglich, die Bohrschablone sicher und zuverlässig an einen zu untersuchenden Holzkörper zu befestigen.

Alternativ ist es auch möglich, die Bohrschablone mit Nägeln oder mit Schrauben an dem zu untersuchenden Holzkörper zu befestigen. In vorteilhafter Weise kann die Bohrschablone, insbesondere an ihren Ecken und/oder Rändern, Bohrungen aufweisen, durch die jeweils eine Befestigungsschraube oder ein Nagel geführt werden kann. Eine Befestigung beispielsweise mit einem Spanngurt oder mit mehreren Spanngurten hat jedoch den besonderen Vorteil, dass die Bohrschablone nach Erfolg der Untersuchung einfach und beschädigungsfrei wieder entfernt und für die Untersuchung eines weiteren Holzkörpers wieder verwendet werden kann.

Für den Fall, dass der Holzkörper als kreiszylinderförmiger Pfahl ausgebildet ist, ist der Justiervorsatz so geformt, dass eine Bohrnadel des Bohrwiderstandsmessgeräts, insbesondere auch während eines Bohrvorganges, radial zu dem kreiszylinderförmigen Objekt ausgerichtet ist, solange die Anlagepunkte mit dem kreiszylinderförmigen Objekt direkt oder indirekt in Kontakt stehen.

Vorzugsweise ist der Justiervorsatz derart ausgebildet, dass der Benutzer das Bohrwiderstandsmessgerät nicht versehentlich relativ zu dem Holzkörper verkippen kann, solange die Anlagepunkte des Justiervorsatzes an dem Holzkörper direkt oder indirekt an liegen. Hierbei ist es insbesondere auch möglich, die Anlagepunkte des Justiervorsatzes in Kontakt mit der an dem Holzkörper befestigten Bohrschablone zu bringen, um eine korrekte Ausrichtung des Bohrwiderstandsmessgerätes zu bewirken.

Bei Verwendung einer Bohrschablone wird das Bohrwiderstandsmessgerät vorzugsweise derart platziert, dass die Bohrnadel bei der Durchführung einer Untersuchungsbohrung durch einen der Durchbrüche, die die Anfänge der Bohrkanäle definieren, verläuft.

Um dies zu gewährleisten weist die Bohrschablone erfindungsgemäß wenigstens ein Justierelement auf, das mit dem Justiervorsatz des Bohrwiderstandsmessgerätes zusammen wirkt. Beispielsweise kann das Justierelement als Justierbohrung ausgebildet sein, in die eine Justierspitze, die einen der Anlagepunkte aufweist, eingreift. Vorzugsweise ist das Justierelement genau einem Durchbruch für die Bohrnadel zugeordnet. Insbesondere kann vorteilhaft vorgesehen sein, dass jedem Durchbruch der Bohrschablone wenigstens ein Justierelement zugeordnet ist.

Bei einer ganz besonders vorteilhaften Ausführung sind jedem Durchbruch der Bohrschablone jeweils mehrere Justierelemente, insbesondere Justierbohrungen, zugeordnet, so dass sichergestellt ist, dass die Bohrnadel des Bohrwiderstandsmessgerätes jeweils genau diesen Durchbruch ausgerichtet ist, wenn die Anlagepunkte mit den Justierelementen dieses Durchbruchs zusammen wirken. Beispielsweise können die Justierbohrungen in Reihen rechts und links neben einer Reihe von Durchbrüchen angeordnet sein. Es ist alternativ oder zusätzlich auch möglich, dass einer oder mehrere der Durchbrüche zusätzlich als Justierbohrungen fungieren. Insbesondere kann vorteilhaft vorgesehen sein, dass wenigstens einer der Durchbrüche als Justierbohrung seinem unmittelbar benachbarten Durchbruch zugeordnet ist.

Bei einer besonderen Ausführung weist der Justiervorsatz vier Justierspitzen auf, die in Justierbohrungen, die um den jeweiligen zugeordneten Durchbruch herum angeordnet sind, einführbarbar sind. Sobald die Justierspitzen in die Justierbohrungen eingeführt sind, ist die Bohrnadel des Bohrwiderstandsmessgerätes zwangsläufig auf den zugeordneten Durchbruch ausgerichtet, so dass anschließend die Bohrung durch den Durchbruch hindurch erfolgen kann. Außerdem sind die Justierspitzen derart ausgebildet, dass die Bohrnadel in dieser Stellung stets radial zu dem kreiszylinderförmigen Holzkörper ausgerichtet ist.

Durch die gleichzeitige Verwendung einer Bohrschablone und eines Justiervorsatzes ist für jede Untersuchungsbohrung eindeutig sowohl der Beginn des Bohrkanals, als auch die Ausrichtung des Bohrwiderstandsmessgerätes eindeutig festgelegt. Die Gefahr einer falsch ausgerichteten und/oder an der falschen Stelle beginnenden Untersuchungsbohrung ist hierdurch ausgeschlossen.

In der Zeichnung ist der Erfindungsgegenstand beispielhaft und schematisch dargestellt und wird anhand der Figuren nachfolgend beschrieben, wobei gleiche oder gleich wirkende Elemente zumeist mit denselben Bezugszeichen versehen sind. Dabei zeigen:
- Fig. 1: eine schematische Darstellung zur Illustration eines Ausführungsbeispiels des erfindungsgemäßen Verfahrens,
- Fig. 2: ein Ausführungsbeispiel einer erfindungsgemäßen Bohrschablone,
- Fig. 3: ein weiteres Ausführungsbeispiel einer erfindungsgemäßen Bohrschablone,
- Fig. 4: eine Illustration der Verwendung der in Fig. 3 dargestellten Bohrschablone in Kombination mit einem Ausführungsbeispiel eines erfindungsgemäßen Bohrwiderstandsmessgeräts mit Justiervorsatz,
- Fig. 5: eine weitere Illustration der Verwendung eines Ausführungsbeispiels einer erfindungsgemäßen Bohrschablone in Kombination mit einem Ausführungsbeispiel eines erfindungsgemäßen Bohrwiderstandsmessgeräts mit Justiervorsatz in einer Seitenquerschnittsdarstellung, und
- Fig. 6: die Verwendung eines Ausführungsbeispiels einer erfindungsgemäßen Bohrschablone in Kombination mit einem Ausführungsbeispiel eines erfindungsgemäßen Bohrwiderstandsmessgeräts in einer Radialquerschnittsdarstellung.

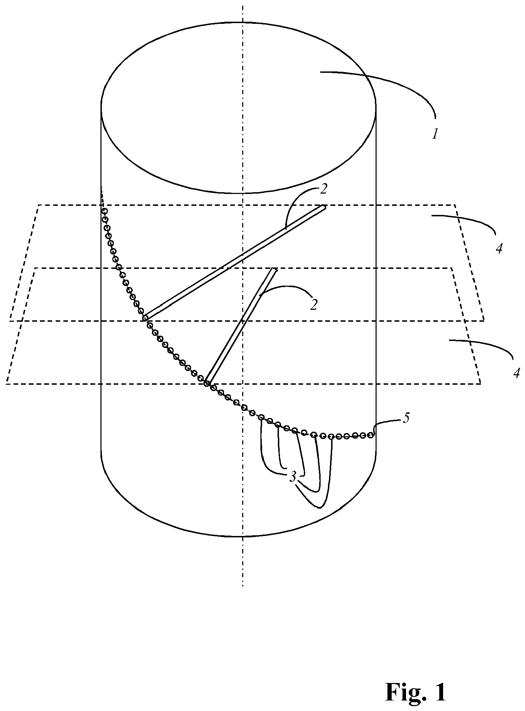

Figur 1 illustriert beispielhaft ein Ausführungsbeispiel eines Verfahrens zum Untersuchen eines, insbesondere unter Wasser befindlichen Holzkörpers 1 auf Befall mit Holzschädlingen. Bei einer solchen Untersuchung spielen Holzkörper, die eine kreiszylinderförmige Form aufweisen, wie beispielsweise Pfähle, insbesondere Dalben, oder Pfosten, eine ganz besondere Rolle, weil diese häufig, insbesondere bei Pfahlgründungen im Wasser, verwendet werden. Aus diesem Grund ist in Figur 1 ein Holzkörper 1 dargestellt, der eine kreiszylinderförmige Form aufweist. Die Erfindung ist jedoch nicht auf derartige Holzkörper beschränkt.

Bei der Durchführung des Verfahrens werden mehrere Untersuchungsbohrungen 2 durchgeführt, bei denen der einer Bohrnadel von dem Holzkörper 1 entgegengesetzte Widerstand in Abhängigkeit von der Eindringtiefe beim Eindringen der Bohrnadel in den Holzkörper 1 und/oder beim Herausziehen der Bohrnadel aus dem Holzkörper 1 gemessen wird. In der Figur sind der besseren Übersichtlichkeit halber lediglich die Anfänge 3 der Bohrkanäle 2 und exemplarisch lediglich 2 Bohrkanäle eingezeichnet.

Die Untersuchungsbohrungen werden derart ausgeführt, dass die Bohrkanäle 2 in unterschiedlichen, zueinander parallelen Ebenen 4 verlaufen. Der besseren Übersichtlichkeit halber sind lediglich zwei der zueinander parallelen Ebenen 4 eingezeichnet.

An dieser Stelle sei darauf hingewiesen, dass der Begriff "Ebene" in dieser Anmeldung nicht für ein strukturelles Objekt gebraucht wird, sondern dazu, die geometrische Anordnung der Bohrkanäle zu beschreiben. Gleiches gilt in Bezug auf den Begriff "Helixkurve".

Bei dem dargestellten Ausführungsbeispiel wird in jeder der zueinander parallelen Ebenen eine einzige Untersuchungsbohrung durchgeführt, was in aller Regel vollkommen ausreichend ist, um einen Befall feststellen zu können und/oder um das Ausmaß eines Befalls feststellen zu können.

Es ist deutlich zu erkennen, dass die Anfänge 3 der Bohrkanäle 2 entlang einer Helixkurve 5 verlaufen.

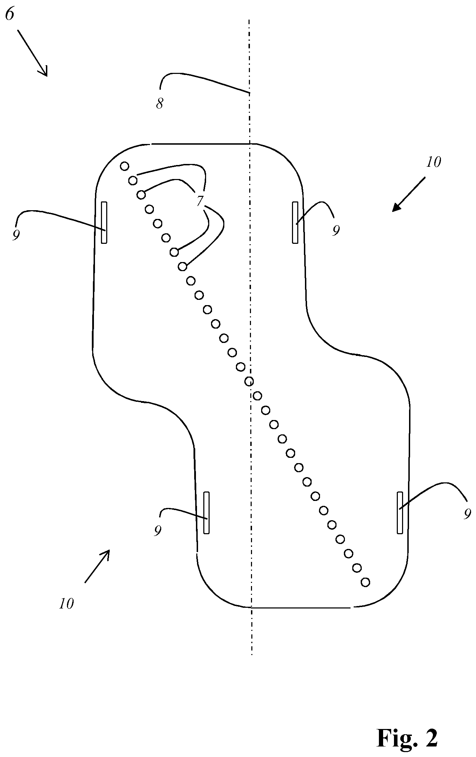

Figur 2 zeigt ein Ausführungsbeispiel einer erfindungsgemäßen Bohrschablone 6, die insbesondere aus einem flachen und elastisch biegbaren Material, beispielsweise aus einem rostfreien Blech oder aus Kunststoff gebildet sein kann. Die Bohrschablone 6 weist Durchgänge 7 auf, die entlang einer geraden Linie (nicht eingezeichnet) angeordnet sind. Wird die Bohrschablone 6 um einen Holzkörper herum gelegt und dabei um eine zur Biegeachse 8 koaxiale oder parallele Achse gebogen, so richten sich die Durchgänge 7 automatische entlang einer Helixkurve aus.

Die Durchgänge 7 definieren die Anfänge der Bohrkanäle 2. Die die Untersuchung durchführende Person muss die Bohrnadel lediglich durch jeweils einen der Durchgänge 7 hindurch stecken und anschließend die Untersuchungsbohrung durchführen, wobei die Bohrnadel in Radialrichtung des Holzkörpers 1 bewegt wird. Vorzugsweise wird die Bohrnadel 15 senkrecht zur Oberfläche der Bohrschablone 6 und/oder des Holzkörpers 1 angesetzt. Die Bohrschablone 6 weist darüber hinaus Schlitzdurchbrüche 9 auf, durch die ein Spanngurt oder ein elastischer Gurt (nicht dargestellt) gezogen werden kann, um die Bohrschablone 6 an einem zu untersuchenden Holzkörper 1 befestigen zu können.

Außerdem weist die Bohrschablone 6 Aussparungen 10 auf, die derart angeordnet sind, dass die Spitze der Bohrnadel 15 die Bohrschablone 6 nicht beschädigen können, wenn sie auf der jeweils gegenüberliegenden Seite des Holzkörpers 1 aus dem Holzkörper 1 austritt.

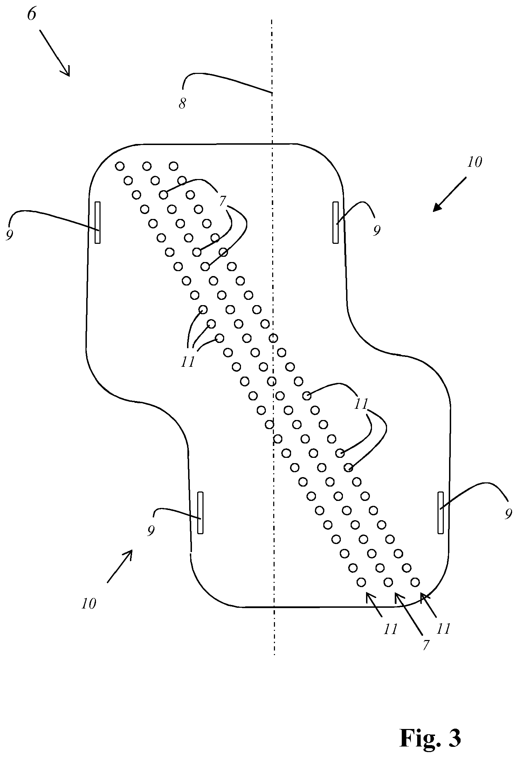

Figur 3 zeigt ein weiteres Ausführungsbeispiel einer erfindungsgemäßen Bohrschablone 6, die weitgehend genauso aufgebaut ist, wie die in Figur 2 dargestellte Bohrschablone 6. Auch die in Figur 3 gezeigte Bohrschablone 6 weist Durchbrüche 7 auf, die entlang einer geraden Linie angeordnet sind.

Rechts und links dieser Linie von Durchbrüchen 7 befindet sich jeweils eine Linie von Justierbohrungen 11, die dazu ausgebildet und bestimmt sind, mit einem besonderen Justiervorsatz 12 eines Bohrwiderstandsmessgeräts 14 zusammenzuwirken.

Jedem Durchbruch 7 sind zwei Justierbohrungen 11 zugeordnet. Es ist bei diesem Ausführungsbeispiel vorgesehen, dass die Bohrschablone 6 zusammen mit einem Justiervorsatz 12 eines Bohrwiderstandsmessgeräts 14 eingesetzt wird, die vier Justierspitzen 13 aufweist.

Hierzu wird die Bohrschablone 6 zunächst an einem kreiszylinderförmigen Holzkörper 16 befestigt. Wie weiter oben bereits beschrieben ist, sind die Durchbrüche 7 entlang einer um den Holzkörper 1 herum verlaufenden Helixkurve angeordnet. In einem nächsten Schritt wird das Bohrwiderstandsmessgerät angelegt. Hierbei greifen zwei der Justierspitzen 13 in die dem jeweiligen Durchbruch 7 zugeordneten Justierbohrungen 11 ein. Die beiden anderen der vier Justierspitzen 13 greifen in die benachbarten Durchbrüche 7, die auf diese Weise zusätzlich als Justierbohrungen dienen, ein.

Jede Justierspitze 13 weist einen Anlagepunkt auf, der mit dem Holzkörper 1 in Kontakt steht.

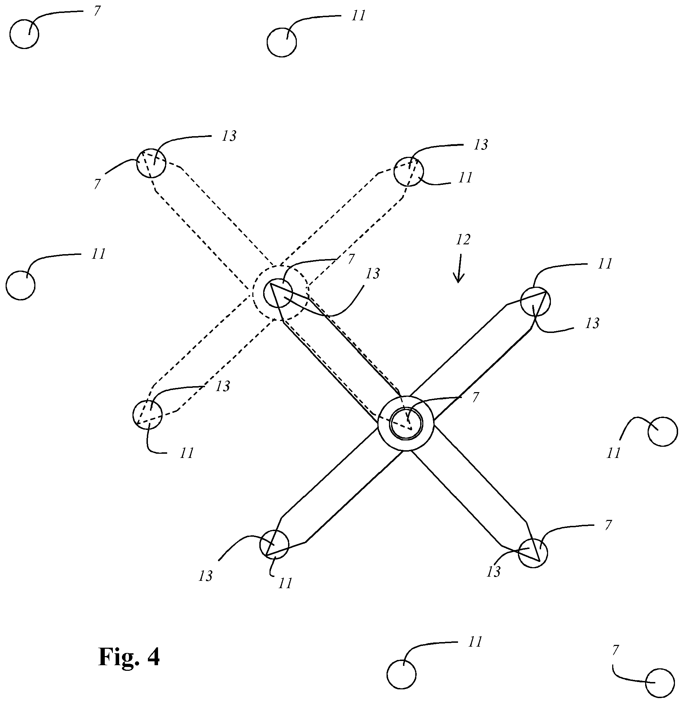

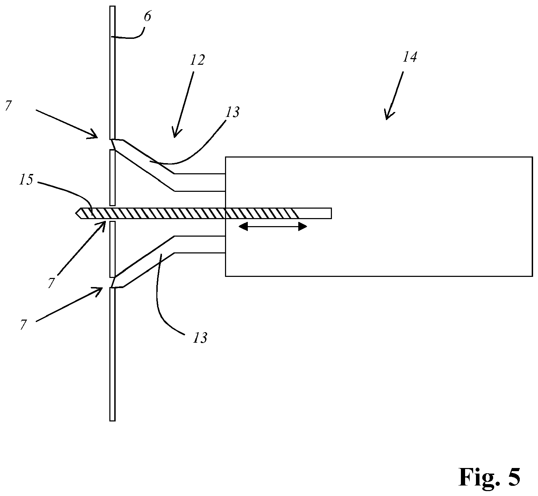

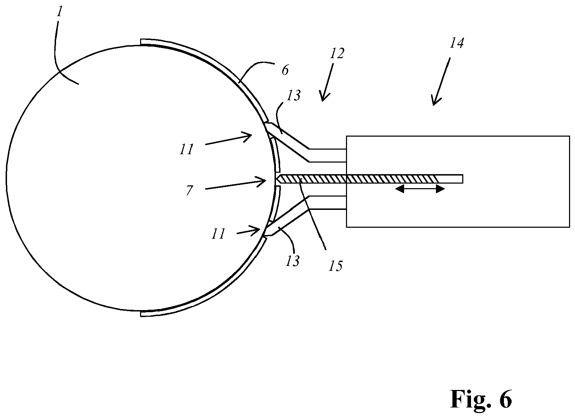

Der Justiervorsatz 12, die Durchbrüche 7 sowie die Justierbohrungen 11 sind derart ausgebildet und angeordnet, dass die Bohrnadel 15 des Bohrwiderstandsmessgeräts 14 bei einem Bohrvorgang genau durch den Durchbruch 7 geführt wird, der den beiden Justierbohrungen 11 zugeordnet ist, was in den Figuren 4, 5 und 6 im Detail illustriert ist. Darüber sind Justiervorsatz 12, die Durchbrüche 7 sowie die Justierbohrungen 11 derart ausgebildet und angeordnet, dass die Bohrnadel 15 des Bohrwiderstandsmessgeräts 14 stets radial zu dem kreiszylinderförmigen Holzkörper 1 ausgerichtet ist. Auch dies ist in den Figuren 4, 5 und 6 zu erkennen.

Das in dieser Weise angesetzte Bohrwiderstandsmessgerät 14 weist durch das Zusammenwirken der Bohrschablone 6 mit dem Justiervorsatz 12 relativ zu dem Holzkörper 1 eine genau definierte und eindeutige Position, sowie eine genau definierte und eindeutige Ausrichtung, nämlich eine radiale Ausrichtung, auf. Das auf diese Weise angesetzte Bohrwiderstandsmessgerät 14 kann zur Durchführung der Untersuchungsbohrung nun seine Bohrnadel 15, insbesondere motorisch angetrieben, drehend in den Holzkörper 1 hineintreiben, wobei sichergestellt ist, dass der Bohrkanal 15 die gewünschte Position und Ausrichtung hat.

Nach Durchführung einer Untersuchungsbohrung wird das Bohrwiderstandsmessgerät 14 abgenommen und erneut derart an die Bohrschablone 6 angekoppelt, dass die nächste Untersuchungsbohrung durch den nächsten Durchbruch 7, der zuvor noch als Justierbohrung 11 fungiert hat, erfolgen kann. Der für die nächste Untersuchungsbohrung angesetzte Justiervorsatz 12 ist in Figur 4 gestrichelt eingezeichnet. Die beiden Justierspitzen 13, die bei der vorausgegangenen Untersuchungsbohrung in Justierbohrungen 11 eingegriffen haben, greifen hierbei in die nächsten Justierbohrungen 11 ein, die den vorherig benutzen Justierbohrungen 11 unmittelbar benachbart sind, während die beiden anderen Justierspitzen 13 in die nächsten Durchbrüche 7 eingreifen, die dann als Justierbohrungen 11 dienen.

### Bezugszeichenliste:

- 1: Holzkörper
- 2: Bohrkanäle
- 3: Anfänge der Bohrkanäle 2
- 4: Ebenen, in denen die Bohrkanäle angeordnet sind
- 5: Helixkurve
- 6: Bohrschablone
- 7: Durchbrüche
- 8: Biegeachse
- 9: Schlitzdurchbrüche
- 10: Aussparungen
- 11: Justierbohrung
- 12: Justiervorsatz
- 13: Justierspitzen
- 14: Bohrwiderstandsmessgerät
- 15: Bohrnadel

## Patentansprüche

1. Verfahren zum Untersuchen eines unter Wasser befindlichen länglichen Holzkörpers (1) auf Befall mit Holzschädlingen, insbesondere im Meer lebender Holzschädlinge, und/oder zum Ermitteln eines Ausmaßes eines Befalls mit Holzschädlingen, insbesondere im Meer lebender Holzschädlinge, an einem Holzkörper (1), **dadurch gekennzeichnet, dass** mit einem Bohrwiederstandmessgerät (14) mehrere Untersuchungsbohrungen durchgeführt werden, bei denen der einer Bohrnadel (15) von dem Holzkörper (1) entgegengesetzte Widerstand in Abhängigkeit von der Eindringtiefe beim Eindringen der Bohrnadel (15) in den Holzkörper (1) und/oder beim Herausziehen der Bohrnadel (15) aus dem Holzkörper (1) gemessen wird, und dass die Bohrkanäle (2) in unterschiedlichen zueinander parallelen Ebenen (4) Verlaufen, wobei die Ebenen (4) senkrecht zu einer Längserstreckungsachse des länglichen Holzkörpers (1) angeordnet sind, und dass
a. die Untersuchungsbohrungen bei einem kreiszylinderförmigen oder halbkreiszylinderförmigen Holzkörper (1) derart durchgeführt werden, dass die Anfänge der Bohrkanäle entlang einer Helixkurve (5) angeordnet sind, und/oder dass
b. eine Bohrschablone (6) an dem Holzkörper (1) befestigt wird, die die Anfänge der Bohrkanäle (2) definiert, wobei die Bohrschablone (6) ein Justierelement aufweist, das mit einem Justiervorsatz (12) des Bohrwiederstandmessgerätes (14) zusammen wirkt.

2. Verfahren nach Anspruch 1, **dadurch gekennzeichnet, dass** die Untersuchungsbohrungen derart ausgeführt werden, dass die Bohrkanäle (2) vollständig durch den Holzkörper (1) hindurch verlaufen.

3. Verfahren nach Anspruch 1 oder 2, **dadurch gekennzeichnet, dass** die Untersuchungsbohrungen derart ausgeführt werden, dass die Abstände der Anfänge benachbarter Bohrkanäle (2) gleich sind.

4. Verfahren nach einem der Ansprüche 1 bis 3, **dadurch gekennzeichnet, dass** die Untersuchungsbohrungen derart ausgeführt werden, dass die Anfänge benachbarter Bohrkanäle (2) einen Abstand zueinander aufweisen, der im Bereich des halben bis doppelten Durchmessers, insbesondere im Bereich des halben bis ganzen Durchmessers, der Bohrnadel (15) liegt.

5. Verfahren nach einem der Ansprüche 1 bis 4, **dadurch gekennzeichnet, dass** die Untersuchungsbohrungen derart ausgeführt werden, dass die Anfänge benachbarter Bohrkanäle (2) einen Abstand zueinander aufweisen, der im Bereich von 0,5 cm bis 1,5 cm liegt.

6. Verfahren zum Untersuchen eines unter Wasser, insbesondere im Meer, befindlichen, insbesondere länglichen, Holzkörpers (1) auf Befall mit Holzschädlingen, insbesondere im Meer lebender Holzschädlinge, **dadurch gekennzeichnet, dass** zunächst eine Untersuchung, nach einem Verfahren nach einem der Ansprüche 1 bis 5, durchgeführt wird und anschließend ein Opferholzkörper unter Wasser an dem Holzkörper (1) befestigt wird, der später einmalig oder in regelmäßigen Zeitabständen auf Befall untersucht wird.

7. Verfahren nach Anspruch 6, **dadurch gekennzeichnet, dass** bei späteren Untersuchungen der jeweils an dem Holzkörper (1) befestigte Opferholzkörper demontiert und jeweils durch einen weiteren, neuen Opferholzkörper ersetzt wird.

8. Verfahren nach einem der Ansprüche 1 bis 7, **dadurch kennzeichnet, dass** die Bohrschablone (6) flach und elastisch biegbar ausgebildet ist.

9. Verfahren nach einem der Ansprüche 1 bis 8, **dadurch kennzeichnet, dass** die Bohrschablone (6) um einen zu untersuchenden Holzkörper, insbesondere flächig anliegend, herum gelegt wird, wobei die Bohrschablone (6) für einen Holzkörper (1) mit dem Umfang x ausgelegt ist und die Breite der Bohrschablone (6) wenigstens x/2 beträgt.

10. Verfahren nach Anspruch 9, **dadurch kennzeichnet, dass** die Bohrschablone (6) eine Vielzahl von Durchbrüchen (7) Aufweist, durch die hindurch eine Bohrnadel (15) führbar ist und dass die Bohrschablone (6) wenigstens eine Aussparung (10) aufweist, die wenigstens einem der Durchbrüche (7) in Bohrrichtung gesehen gegenüberliegend angeordnet ist, wenn die Bohrschablone (6) um einen Holzkörper (1) herum gelegt ist.

11. Set zur Verwendung bei der Ausführung eines Verfahrens nach einem der Ansprüche 1 bis 10 mit einem Bohrwiderstandsmessgerät (14), das eine Bohrnadel (15) aufweist und mit einem Justiervorsatz (12) zum Positionieren und/oder Ausrichten des Bohrwiderstandsmessgeräts (14) relativ zu einem zu untersuchenden Holzkörper (1), und mit einer Bohrschablone (6), die ein Justierelement aufweist, wobei der Justiervorsatz (12) dazu ausgebildet ist, mit dem Justierelement der Bohrschablone (6) zusammen zu wirken.

## Claims

1. Method for examining an elongate wood body (1) located under water for infestation with wood pests, in particular wood pests living in the sea, and/or for determining an extent of an infestation with wood pests, in particular wood pests living in the sea, on a wood body (1), **characterized in that** a plurality of investigative drillings are carried out by means of a drilling resistance measuring instrument (14), during which the resistance to which a drilling needle (15) is subjected by the wood body (1) is measured in dependence on the penetration depth when the drilling needle (15) penetrates into the wood body (1) and/or when the drilling needle (15) is pulled out of the wood body (1), and **in that** the drilled channels (2) run in different, mutually parallel planes (4), wherein the planes (4) are arranged perpendicular to an axis of longitudinal extent of the elongate wood body (1), and **in that**
a. in the case of a circular-cylindrical or semicircular-cylindrical wood body (1), the investigative drillings are carried out in such a manner that the beginnings of the drilled channels are arranged along a helix curve (5), and/or **in that**
b. a drilling template (6) is fastened to the wood body (1) and defines the beginnings of the drilled channels (2), wherein the drilling template (6) has an alignment element which interacts with an alignment attachment (12) of the drilling resistance measuring instrument (14) .

2. Method according to Claim 1, **characterized in that** the investigative drillings are performed in such a manner that the drilled channels (2) run completely through the wood body (1).

3. Method according to Claim 1 or 2, **characterized in that** the investigative drillings are performed in such a manner that the spacings between the beginnings of neighbouring drilled channels (2) are the same.

4. Method according to one of Claims 1 to 3, **characterized in that** the investigative drillings are performed in such a manner that the beginnings of neighbouring drilled channels (2) have a spacing with respect to one another which is in the range of half of the diameter to double the diameter of the drilling needle (15), in particular in the range of half of the diameter to the full diameter of the drilling needle (15).

5. Method according to one of Claims 1 to 4, **characterized in that** the investigative drillings are performed in such a manner that the beginnings of neighbouring drilled channels (2) have a spacing with respect to one another which is in the range from 0.5 cm to 1.5 cm.

6. Method for examining an in particular elongate wood body (1) located under water, in particular located in the sea, for infestation with wood pests, in particular wood pests living in the sea, **characterized in that** firstly an examination is carried out by a method according to one of Claims 1 to 5, and then a sacrificial wood body is fastened under water to the wood body (1) and is later examined, once or at regular time intervals, for infestation.

7. Method according to Claim 6, **characterized in that**, during later examinations, the sacrificial wood body respectively fastened to the wood body (1) is removed and replaced in each case by another, new sacrificial wood body.

8. Method according to one of Claims 1 to 7, **characterized in that** the drilling template (6) has a flat and elastically flexible form.

9. Method according to one of Claims 1 to 8, **characterized in that** the drilling template (6) is laid around, in particular in a manner lying flat against, a wood body to be examined, wherein the drilling template (6) is configured for a wood body (1) with the circumference x and the width of the drilling template (6) amounts to at least x/2.

10. Method according to Claim 9, **characterized in that** the drilling template (6) has a multiplicity of apertures (7) through which a drilling needle (15) can be guided, and **in that** the drilling template (6) has at least one cutout (10) which is arranged opposite at least one of the apertures (7), as viewed in the drilling direction, when the drilling template (6) is laid around a wood body (1).

11. Set for use in performing a method according to one of Claims 1 to 10, with a drilling resistance measuring instrument (14) which has a drilling needle (15), and with an alignment attachment (12) for positioning and/or orienting the drilling resistance measuring instrument (14) relative to a wood body (1) to be examined, and with a drilling template (6) which has an alignment element, wherein the alignment attachment (12) is designed to interact with the alignment element of the drilling template (6).

## Revendications

1. Procédé d'analyse d'un corps en bois allongé (1) se trouvant sous l'eau afin de déceler une infestation par des parasites du bois, en particulier des parasites du bois vivant dans la mer, et/ou pour déterminer une mesure d'une infestation avec des parasites du bois, en particulier des parasites du bois vivant dans la mer, sur un corps en bois (1), **caractérisé en ce que** plusieurs perçages d'analyse sont effectués avec un appareil de mesure de résistance au perçage (14), dans lesquels la résistance opposée par le corps en bois (1) à une aiguille de perçage (15) est mesurée en fonction de la profondeur de pénétration lors de l'enfoncement de l'aiguille de perçage (15) dans le corps en bois (1) et/ou lors du retrait de l'aiguille de perçage (15) hors du corps en bois (1), et **en ce que** les canaux de perçage (2) s'étendent dans des plans parallèles (4) différents les uns des autres, les plans (4) étant disposés perpendiculairement à un axe d'étendue longitudinale du corps en bois allongé (1) et **en ce que**
a. les perçages d'analyse, dans le cas d'un corps en bois (1) de forme cylindrique circulaire ou semi-cylindrique circulaire, sont réalisés de telle sorte que les débuts des canaux de perçage soient disposés le long d'une courbe hélicoïdale (5) et/ou **en ce que**
b. un gabarit de perçage (6) est fixé au corps en bois (1), lequel définit les débuts des canaux de perçage (2), le gabarit de perçage (6) présentant un élément d'ajustement qui coopère avec une tête d'ajustement (12) de l'appareil de mesure de résistance au perçage (14).

2. Procédé selon la revendication 1, **caractérisé en ce que** les perçages d'analyse sont réalisés de telle sorte que les canaux de perçage (2) s'étendent complètement à travers le corps en bois (1) .

3. Procédé selon la revendication 1 ou 2, **caractérisé en ce que** les perçages d'analyse sont réalisés de telle sorte que les distances entre les débuts de canaux de perçage adjacents (2) soient identiques.

4. Procédé selon l'une quelconque des revendications 1 à 3, **caractérisé en ce que** les perçages d'analyse sont réalisés de telle sorte que les débuts de canaux de perçage adjacents (2) soient espacés les uns des autres d'une distance qui est située dans la plage de la moitié au double du diamètre, en particulier dans la plage de la moitié au diamètre total de l'aiguille de perçage (15) .

5. Procédé selon l'une quelconque des revendications 1 à 4, **caractérisé en ce que** les perçages d'analyse sont réalisés de telle sorte que les débuts de canaux de perçage adjacents (2) soient espacés les uns des autres d'une distance qui est située dans une plage de 0,5 cm à 1,5 cm.

6. Procédé d'analyse d'un corps en bois en particulier allongé (1) se trouvant sous l'eau, en particulier dans la mer, afin de déceler une infestation par des parasites du bois, en particulier des parasites du bois vivant dans la mer, **caractérisé en ce que** l'on effectue tout d'abord une analyse selon un procédé selon l'une quelconque des revendications 1 à 5, puis un corps en bois sacrificiel est fixé sous l'eau au corps en bois (1), lequel sera analysé ultérieurement une fois ou à intervalles réguliers pour déceler une infestation.

7. Procédé selon la revendication 6, **caractérisé en ce que** lors d'analyses ultérieures, le corps en bois sacrificiel fixé dans chaque cas au corps en bois (1) est démonté et remplacé à chaque fois par un nouveau corps en bois sacrificiel supplémentaire.

8. Procédé selon l'une quelconque des revendications 1 à 7, **caractérisé en ce que** le gabarit de perçage (6) est réalisé sous forme plate et élastiquement flexible.

9. Procédé selon l'une quelconque des revendications 1 à 8, **caractérisé en ce que** le gabarit de perçage (6) est placé autour d'un corps en bois à analyser, en particulier à plat contre celui-ci, le gabarit de perçage (6) étant conçu pour un corps en bois (1) de circonférence x et la largeur du gabarit de perçage (6) étant d'au moins x/2.

10. Procédé selon la revendication 9, **caractérisé en ce que** le gabarit de perçage (6) présente une pluralité d'orifices (7) à travers lesquels peut être guidée une aiguille de perçage (15) et **en ce que** le gabarit de perçage (6) présente au moins un évidement (10) qui est disposé au moins à l'opposé de l'un des orifices (7), vu dans la direction de perçage, lorsque le gabarit de perçage (6) est placé autour d'un corps en bois (1).

11. Ensemble pour l'utilisation pour mettre en œuvre un procédé selon l'une quelconque des revendications 1 à 10, comprenant un appareil de mesure de résistance au perçage (14) qui présente une aiguille de perçage (15) et comprenant une tête d'ajustement (12) pour le positionnement et/ou l'orientation de l'appareil de mesure de résistance au perçage (14) par rapport à un corps en bois (1) à analyser, et comprenant un gabarit de perçage (6) qui présente un élément d'ajustement, la tête d'ajustement (12) étant réalisée de manière à coopérer avec l'élément d'ajustement du gabarit de perçage (6).
